# NEW EUROPEAN PATENT SPECIFICATION

(11) **EP 2 569 165 B2**
(45) Date of publication and mention of the opposition decision: **18.01.2023**
(45) Mention of the grant of the patent: 26.08.2015
(21) Application number: 11724441.8
(22) Date of filing: 11.05.2011
(51) Int. Cl.: B41J 11/00, B41M 7/00, B42D 25/29, D21H 21/36, D21H 19/10, A61L 2/18, C09D 5/14

(54) **A METHOD OF PRINTING**
VERFAHREN ZUM DRUCKEN
PROCÉDÉ D'IMPRESSION

(30) Priority: 11.05.2010 GB 201007853
(43) Date of publication of application: 20.03.2013
(60) Divisional application: 15172801.1
(73) Proprietor: HeiQ Materials AG, 8952 Schlieren (CH)
(72) Inventor: HINTON-LEVER, Andrew, Huddersfield, Yorkshire HD7 5BG (GB); DUGDALE, Julian, Huddersfield, Yorkshire HD7 5BG (GB)
(74) Representative: Bremi, Tobias Hans
(86) International application number: PCT/GB2011/050905
(87) International publication number: WO 2011/141740

(56) References cited:
- EP-A1- 0 749 848
- EP-A1- 1 186 439
- WO-A1-2008/097314
- WO-A1-2010/025350
- GB-A- 2 324 801
- JP-A- 9 001 952
- JP-A- 9 066 684
- JP-A- 9 175 061
- JP-A- H0 939 369
- US-A- 5 962 137
- US-A1- 2002 058 111
- US-A1- 2004 084 163
- "pages 20-50, 362-365, and 475-476" In: R.H. LEACH: "The Printing Ink Manual", 1988, Blueprint, London pages 20-50,362ff,
- Manroland 505LV, Printweek, 27 May 2009
- Manroland 505LV, Printweek hard copy version, 29 May 2009
- Roland 505LV - website extract from:http://www.manrolandbenelux be/layout1 php?lid=3&pr=289&sm=431 &u=11 &p=831
- Roland R 505 0B LV - advertisement providing press specifications
- Marketing literature for the Lithrone S26/S29 (published in March 2009)
- Komori: "Lithrone S26(P) and Lithrone S29(P)", Press release, 17 February 2006 (2006-02-17),

## Description

This invention relates to a printing method.

Printed matter, for example, documents, bank notes, books and magazines, advertising material and so on is intended to be held and manipulated by hand. However, printed matter is often intended to be handled by more than one person, and in those circumstances, germs and bacteria can be transferred from one person to the next via the printed matter. The transfer of germs and bacteria, or cross-infection, is generally considered to be undesirable as it can be detrimental to health.

Many institutions and workplaces, such as hospitals, banks, schools, shops, offices and restaurants, have cleaning and cross-infection control procedures in place. Such procedures generally require regular and thorough cleaning of equipment, utensils, tools etc. Nevertheless, institutions often house a great number of portable documents which are regularly handled by various people, but the documents themselves are not subject to strict anti-cross-contamination procedures. In many cases the documents cannot be cleaned because to do so may damage them. It is therefore unsurprising that even despite strict hygiene procedures being in place, cross-infection incidents continue to occur on a fairly regular basis.

Various attempts have been made to reduce this risk, which have tended to concentrate on the use of antibacterial impregnated papers and/or inks. These solutions have not met with overwhelming commercial success for economic and efficacy reasons. Specifically, antibacterial impregnated papers may require expensive new tooling in their manufacture and since only the surface of the paper comes into contact with the user, the relatively expensive antibacterial agents within the bulk of the material are largely ineffective. To impregnate the bulk of the paper, when only the surface of it comes into contact with users is therefore uneconomic and wasteful. On the other hand, antibacterial impregnated inks have also been shown to be largely ineffective since only a portion of the paper's surface is covered therewith, thereby leaving a larger proportion of the paper unprotected.

Further examples of prior art arrangements are disclosed in EP 0 749 848 A1, JP 9 001052 A, JP 9 066684 A, US 5,962,137, WO 2008/097314 A1, EP 1 186 439 A1, US 2002/058111 A1, GB 2 324 801 A, JP 9 175061 A, WO 2012/025350 A1.

A need therefore exists for a more effective means of reducing cross-infection as a result of document and packaging handling. An object of this invention is thus to address, reduce and/or eliminate the risks of cross-infection posed by the manual handling of documents and other printed matter.

According to a first aspect of the invention, there is provided a method of printing and finishing on a substrate according to appended claim 1.

Also disclosed, but not forming part of the present invention, the finishing step comprises applying a finishing composition to a transfer surface; and transferring the finishing composition from the transfer surface to the substrate to form the continuous outer protective later; the finishing composition comprising the antibacterial additive.

Also disclosed, but not forming part of the present invention, the transfer surface is the surface of a roller.

According to an embodiment of the invention, the printing step is performed on a first area of the substrate at the same time as the finishing step is performed on a second area of the substrate.

The printing step is preferably carried out using printing techniques, e.g. lithography, thereby making it cost-effective to implement.

According to an embodiment of the invention, the printing step is carried out using any one or more of the group comprising a lithographic process, an analox process, a Heidelberg process, a web offset process and a Tresu process.

The substrate may be any paper or indeed any printable substrate, including, but without limitation to, paper, card, cardboard and sheet polymers.

The antibacterial additive preferably contains silver ions, and the concentration of silver ions in the outer protective layer is preferably sufficient to impart antibacterial activity thereto.

The invention therefore differs from the antibacterial impregnated papers and inks inasmuch as the antibacterial protection is derived from the use of a continuous outer layer. The use of a continuous outer layer also means that the entire surface of the printed matter (i.e. the un-printed regions between the print, and the print itself) is overlaid with an antibacterial layer. It therefore becomes difficult, if not impossible, to touch any unprotected part of the printed matter, thereby significantly reducing the risk of cross-infection.

Silver ion technology is believed to be particularly suited to this application because of its long-lasting antibacterial activity, its longevity and durability.

In a preferred embodiment of the invention, the outer protective layer comprises a varnish comprising silver ions. By only doping the outer layer with silver ions, the materials costs can be significantly reduced, compared to the cost of impregnating the bulk of the paper.

Also disclosed, but not forming part of the present invention is an apparatus comprising a printing stage wherein one or more layers of print are deposited onto one or more sides of a substrate; and a finishing stage wherein a substantially continuous outer protective layer is applied to the entire surface of one or more sides of the substrate.

Also disclosed, but not forming part of the present invention is, the finishing stage comprises a transfer surface for receiving a finishing composition and for transferring the finishing composition to the substrate.

Also disclosed, but not forming part of the present invention, the transfer surface is a roller.

Also disclosed, but not forming part of the present invention is, the finishing composition comprises an antibacterial additive.

According to the first aspect of the invention, the finishing composition comprises an adhesion agent to facilitate adhesion of the composition to a substrate.

According to the first aspect of the invention, the finishing composition comprises an aqueous or organic solution of silver ions, or preferably a hydrosol/organosol suspension of silver ion particles.

According to an embodiment of the invention, the adhesion agent comprises any one or more of the group comprising a natural resin, a synthetic resin, rosin, a polymer, a monomer, a binder, a film former, an adhesive, a top coat, a varnish, acrylic, modified oil, linseed oil, polyurethane, protein resin, shellac, acrylate and epoxy.

According to a further embodiment, the method of printing comprises a solvent.

According to a further embodiment the solvent comprises any one or more of the group comprising water, alcohol, an ester, vegetable oil and a petroleum distillate.

According to a further embodiment the finishing composition comprises any one or more additives from the group comprising a metal, a matting agent, a wax, a gloss agent, a surface modifier, a pigment, a dye, a dryer, a cross linker, an accelerator and an anti-slip agent.

According to a further embodiment, the finishing composition comprises a 0.1% concentration of silver additive powder in an oil-based coating.

According to a further embodiment, the finishing composition comprises a 1.0% concentration of silver additive powder in a water-based coating.

According to an embodiment of the first or second aspect of the invention, the antibacterial activity meets or exceeds the requirements of JIS Z2801/ISO22196:2007 for MRSA and E.coli.

Figure 1 is a schematic diagram showing a manufacturing process for printed matter, the finishing step not being in accordance with the invention.
Figure 2 is a schematic diagram showing another manufacturing process for printed matter in accordance with the invention.

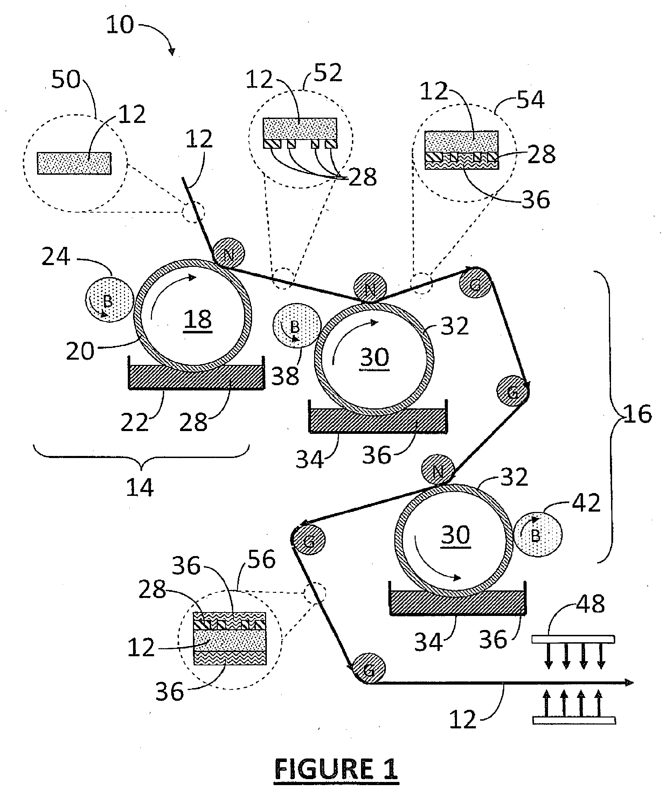

In Figure 1, a lithographic printing press 10 comprises a series of rollers arranged to guide and print onto a continuous web of paper 12. The paper web 12 generally travels from top left to bottom right in the diagram, contacting various rollers in turn. Rollers designated N are nip rollers, those designated G are guide rollers and those designated B are blanket rollers. The printing process comprises two main stages, namely a printing stage 14 and a finishing stage 16.

The printing portion 14 of the press 10 comprises a cylindrical print roller 18, which is arranged to rotate about its longitudinal axis. A printing plate 20, comprising non-printing (negative); hydrophilic (lipophobic) areas and printing (positive), hydrophobic (lipophilic) areas, is wrapped around, and bonded to, the cylindrical print roller 18.

During rotation of the print roller 18, the surface of the printing plate 20 passes through a bath 22 of liquid printing composition 28. The liquid printing composition 28 comprises oil-based ink and a water phase and selectively coats the printing plate 20: the oil-based printing ink 28 adhering to the positive printing areas of the printing plate; and the negative, non-printing areas of the printing plate being protected from ink 28 by the water phase.

As the print roller 18 rotates, it contacts a first blanket roller 24 which squeezes off water on the non-printing, negative area of the printing plate 20. This step prevents the water from wetting the paper 12.

A web of plain, unprinted paper 12, as shown in cross-section in inset 50, is fed into the press 10 from a roll (not shown) of stock and passes between the surface of the print roller 18 and a nip roller N. In this step, an impression of the ink 28 on the print roller 18 is thus transferred onto one side of the paper web 12, as shown in inset 52. The paper web 12 then progresses through the press 10 into the finishing stage 16.

The finishing stage 16 operates in a similar manner to the printing stage 14. A takeup roller 30 having a hydrophobic surface 32, which passes through a bath 34 of oil-based finishing composition 36. The finishing composition 36 comprises an oil-based carrier and a suspension 0.1% suspension of silver ions, which coats the entire surface 30 of the take up roller 30. Excess finishing composition 36 is removed by a second blanket roller 38.

Meanwhile, the web of paper 12 is directed between the take up roller 30 and a second nip roller 40 thereby transferring a layer the finishing composition 36 over the entire surface of the paper web 12, and overlying the previously-deposited ink 28, as shown in inset 54.

The paper web 12 is then passes through a second finishing stage, which coats the opposite side of the web 12. The second finishing stage also comprises a take up roller 30 having a hydrophobic surface 32, which passes through a bath 34 of oil-based finishing composition 36. Again, the finishing composition 36 comprises an oil-based carrier and a suspension 0.1% suspension of silver ions, which coats the entire surface 30 of the take up roller 30. Excess finishing composition 36 is removed by a third blanket roller 42. The web of paper 12 is directed between the second take up roller 30 and a further nip roller 46 thereby transferring a layer the finishing composition 36 over the entire surface of the opposite side of the paper web 12, as shown in inset 56.

Finally, the paper web 12 is passed through a dryer 48 to cure/dry the ink and finishing composition. The drying step 48 may comprise the application of any one or a combination of heat and/or infra red radiation and/or ultra violet radiation. Depending on the type of ink used, the drying process initiates and drives reactive curing of the ink, and/or accelerates absorption and drying.

It will be appreciated that the printing process could be adapted to include any number of printing and/or finishing stages, provided that the final outermost layer deposited on the web is continuous and comprises antibacterial additives.

In a preferred embodiment, the finishing composition 36 comprises: a resin or varnish, whose primary function is to facilitate adhesion of the ink to the paper; a solvent whose primary functions are to act as a carrier for the silver ions; and a silver ion-containing additive, which gives the coating its antibacterial properties. Alternatively, the finishing composition could comprise an equivalent water-based suspension of silver ions. The material (silver ions) is added generally in a liquid form and a carrier liquid is used in most cases (but not exclusively) this would be ethanol based dispersion as supplied. This would be either stirred into or by way of recirculation pump incorporated into the varnish finishing material. This is specific to the water based finishes for oil based finishes the silver ion is supplied as a powder form and is either milled or stirred into the varnish dependant on required particle size.

The resin/varnish can be any natural or synthetic resin, rosin, polymer or monomer, or blend thereof, which acts as a binder, film former, adhesive, top coat, varnish or similar, such as acrylic, modified oil, linseed oil, polyurethane, protein resin, shellac, acrylate, epoxy. The carrier solvent can be any compound or mixture of compounds, generally liquid, designed to allow the printing process to occur and then cease to interact with the coating by reacting, evaporating, absorption, dissipating, becoming sterically hindered/encapsulated, or similar. Examples of commonly used solvents are water, alcohols, esters, vegetable oils and petroleum distillates. Additional additives may be provided, to modify the coating's luster, texture etc., e.g. metals, matting agents, waxes, gloss agents, surface modifiers, pigments, dyes, driers, cross linkers, accelerators and anti-slip agents.

In an embodiment of the invention, a water-based protective outer layer can be applied using a doctor blade and chamber system (i.e. Tresu). In such a situation, the protective outer layer is pumped into interior chamber of an analox roller in liquid form, which liquid fills the cells on the analox cylinder. A doctor blade or (or doctor blades) can be used to skim off any excess coating. The protective coating in the cells can then be transferred to the blanket roller and then transferred onto the printed paper just like in conventional coating.

The protective outer layer comprises a curable/solidiflable liquid (oil or water based) containing an additive comprising silver ions. The concentration of silver ions is sufficient to render the surface of the paper inert to bacterial growth on the surface. The coating, when dried/cured is inert. Testing to JIS Z2801/ISO22196:2007 for MRSA and E.coli have shown that the protective outer layer protects to the required levels under the above ISO requirements.

ISO 22196:2007, formerly JIS Z 2801:2000, which is a standard relating to antibacterial activity specifies a method of ovaluating the antibacterial activity of antibacterial-treated plastic products and other non-porous materials including paint films. According to the standard, a value for the antibacterial activity is determined although there is no pass/fail criterion. Testing to ISO 22196:2007 involves applying a bacterial suspension to a test specimen and to control spoclmons, which arc then incubated for 24 hours.

Bacteria on the test and control samples are counted before and after incubation, and using a formula provided in the standard, the log of the difference between the before and after counts is determined to give a measurement of antimicrobial activity. This can also be expressed as percentage kill.

ISO 22196:2007 specifies the use of two bacterial species, namely Staphylococcus aureus and Escherichia coli, however, testing is routinely carried out using other bacteria as well, e.g. MRSA, Pseudomonas, Listeria, Salmonella, Bacillus, Acinebacter etc., in addition to fungi such as Aspergillus and Candida.

In the present case, six 50mm x 50mm samples were coated with the finishing composition for each bacteria/fungus tested. The antibacterial activity of the finishing composition was tested according to a "non-suspended inoculum (NSI)" method, in which the bacteria are applied to the test specimens as a non-suspended inoculum, which is considered to be a more realistic approach for many coatings/surfaces because the test conditions more closely resemble in-use conditions.

The JIS Z2801 (Antimicrobial Surface Test) is designed to quantitatively measure the ability of an antimicrobial surface to kill or inhibit the growth of microorganisms. The test microorganism is prepared, usually by growth in a liquid culture medium. The suspension of test microorganism is standardized by dilution in a nutritive broth (this affords microorganisms the potential to grow during the test). Control and test surfaces are inoculated with microorganisms, in triplicate, and then the microbial inoculum is covered with a thin, sterile film. Covering the inoculum spreads it, prevents it from evaporating, and ensures close contact with the antimicrobial surface. Microbial concentrations are determined at "time zero" by elution followed by dilution and plating. A control is also run to verify that the neutralization/elution method effectively neutralizes the antimicrobial agent in the antimicrobial surface being tested. Inoculated, covered control and antimicrobial test surfaces are allowed to incubate undisturbed in a humid environment for 24 hours and after incubation, microbial concentrations are determined. The reduction of microorganisms relative to initial concentrations and the control surface is calculated.

The JIS Z 2801 test is quantitative and results tend to be reproducible, provided the inoculum does not spill off of the target area after being covered with the thin film. The method also tests for both bacteriostatic (growth-inhibiting) and bactericidal (bacteria-killing) properties. Because the microbial concentrations are standardized, and bacteria are provided with nutrients during the incubation period, the bacteria are provided with ample opportunity to grow if surfaces are not sufficiently antimicrobial. This is in contrast to certain other antimicrobial tests, where microbes are "incubated" in non-nutritive suspensions, which itself may be stressful over long periods. The method stipulates triplicate experimentation, which helps researchers estimate the precision of the individual tests and increases overall experimental accuracy and includes a rating system for the calculated levels of antimicrobial activity observed in test samples, making determinations of antimicrobial activity less discretionary.

However, the JIS Z 2801 method is not necessarily representative of actual surface contamination events. since a relatively dilute liquid microbial inoculum is spread over a considerable surface area, and then is kept wet (usually for a period of 24 hours). In most real situations, however, microbial contaminants dry quickly onto surfaces, which limits the time that an aqueous medium is available to facilitate interaction between the antimicrobial surface and microorganisms. This means that JIS Z 2801 is a "best-case" sort of test for many products.

The compositions that have been shown to exhibit the above antibacterial activity are: 0.1% concentration of silver additive powder in the oil-based coating; and 1.0% concentration of silver additive liquid in the water-based coating. Additives that have been used successfully are commercially available grades of "BIOMASTER AT300"^{™} and "BIOMASTER GC100"^{™}, which contain silver chloride / titanium composite particles (silver chloride in a protective titanium oxide sponge, 1 - 3 microns), and an anionic gel.

Laboratory test results on printed samples according to the invention are shown in table 1 below

**Table 1 - Test results for antibacterial activity.**

| **Specimen** | **Bacteria** | **Concentration before** | **Concentration after 24h** | **Log₁₀** | **% kill** |
|---|---|---|---|---|---|
| WATER-BASED COATING + BIOMASTER AT300 | MRSA | 2.2E+04 | 9.2E+02 | 1.4 | 95.82% |
| Control | E coli | 2.1E+04 | 2.9E+03 | | |
| WATER-BASED COATING + BIOMASTER AT300 | E coli | 2.1E+04 | < 11.11 | ≥ 3.28 | ≥ 99.95% |
| OIL-BASED COATING + BIOMASTER GC100 | E coli | 2.1E+04 | < 11.11 | ≥ 3.28 | ≥ 99.95% |
| CONTROL | MRSA | 2.5E+04 | 1.6E+04 | | |
| OIL-BASED COATING + BIOMASTER GC100 | MRSA | 2.5E+04 | < 11.11 | ≥ 3.3 5 | ≥ 99.96% |
| WATER-BASED COATING + BIOMASTER AT300 | MRSA | 2.2E+04 | 9.2E+02 | 1.4 | 95.82% |

The applications for the coating according to the invention are considerable and include (but not exclusive to) finishes to food packaging, menus, bank notes, hospital paperwork and any paper board finishes that are touched by humans and therefore could transfer by touch infection.

## Claims

1. A method of printing and finishing on a substrate, the method comprising the following steps, carried out in a single pass of the substrate through a printing press:
a printing step wherein one or more layers of print are deposited onto one or both sides of the substrate;
and a finishing step wherein a liquid finishing composition is applied to the entire surface of one or both sides of the substrate using a doctor blade and chamber system in which the liquid finishing composition is pumped to a chamber and fills cells of a transfer roller, a doctor blade serving to skim off excess liquid finishing composition prior to transfer of the liquid finishing composition to the substrate, and is cured or dried to form a continuous outer protective layer upon the substrate, the finishing composition comprising an antibacterial additive which gives the printed articles antibacterial properties.

2. A method of printing and finishing as claimed in claim 1 wherein the printing step is performed on a first area of the substrate at the same time as the finishing step is performed on a second area of the substrate.

3. A method of printing and finishing as claimed in any of claim 1 to claim 2 wherein the printing method is any one or more of the group comprising a lithographic process, an analox process, a web offset process and a Tresu process.

4. A method of printing and finishing as claimed in any of claim 1 to claim 3 in which the finishing composition comprises an adhesion agent to facilitate adhesion of the composition to a substrate.

5. A method of printing and finishing as claimed in any of claim 1 to claim 3 in which the finishing composition comprises an aqueous or organic solution of silver ions.

6. A method of printing and finishing as claimed in any of claim 1 to claim 3 in which the finishing composition comprises a hydrosol or organosol suspension of silver ion particles.

7. A method of printing and finishing as claimed in claim 4 wherein the adhesion agent comprises any one or more of the group comprising a natural resin, a synthetic resin, rosin, a polymer, a monomer, a binder, a film former, an adhesive, a top coat, a varnish, acrylic, modified oil, linseed oil, polyurethane, protein resin, shellac, acrylate and epoxy.

8. A method of printing and finishing as claimed in any of claim 1 to claim 3, in which the finishing composition comprises a solvent.

9. A method of printing and finishing as claimed in claim 8 wherein the solvent comprises any one or more of the group comprising water, alcohol, an ester, vegetable oil and a petroleum distillate.

10. A method of printing and finishing as claimed in any preceding claim in which the finishing composition comprises any one or more additives from the group comprising a metal, a matting agent, a wax, a gloss agent, a surface modifier, a pigment, a dye, a dryer, a cross linker, an accelerator and an anti-slip agent.

11. A method of printing and finishing as claimed in any preceding claim in which the finishing composition comprises a 0.1% con- centration of silver additive powder in an oil-based coating.

12. A method of printing and finishing as claimed in any of claim 1 to claim 10 in which the finishing composition comprises a 1.0% concentration of silver additive liquid in a water-based coating.

13. A method of printing and finishing according to any preceding claim, wherein the antibacterial activity meets or exceeds the requirements of JIS Z2801/1SO22196:2007 for MRSA and E.coli.

14. A method of printing and finishing according to any preceding claim, wherein the substrate comprises any one or more of the group comprising paper, card, cardboard and a polymer.

## Patentansprüche

1. Verfahren zum Bedrucken und Veredeln eines Substrats, wobei das Verfahren die folgenden Schritte umfasst, die in einem einzigen Durchgang des Substrats durch eine Druckpresse ausgeführt werden:
einen Druckschritt, bei dem eine oder mehrere Druckschichten auf eine oder beide Seiten des Substrats aufgebracht werden; und einen Endbearbeitungsschritt, bei dem eine flüssige Endbearbeitungszusammensetzung auf die gesamte Oberfläche einer oder beider Seiten des Substrats unter Verwendung eines Rakel- und Kammersystems aufgetragen wird, in dem die flüssige Endbearbeitungszusammensetzung in eine Kammer gepumpt wird und Zellen einer Übertragungswalze füllt, wobei eine Rakel dazu dient, überschüssige flüssige Endbearbeitungszusammensetzung vor der Übertragung der flüssigen Endbearbeitungszusammensetzung auf das Substrat abzuschöpfen, und
ausgehärtet oder getrocknet wird, um eine kontinuierliche äußere Schutzschicht auf dem Substrat zu bilden, wobei die Endbearbeitungszusammensetzung einen antibakteriellen Zusatzstoff enthält, der den bedruckten Artikeln antibakterielle Eigenschaften verleiht.

2. Verfahren zum Drucken und Veredeln nach Anspruch 1, wobei der Druckschritt auf einer ersten Fläche des Substrats zur gleichen Zeit wie der Veredelungsschritt auf einer zweiten Fläche des Substrats durchgeführt wird.

3. Verfahren zum Drucken und Veredeln nach einem der Ansprüche 1 bis 2, wobei das Druckverfahren eines oder mehrere aus der Gruppe ist, die ein lithografisches Verfahren, ein Analox-Verfahren, ein Rollenoffsetverfahren und ein Tresu-Verfahren umfasst.

4. Verfahren zum Drucken und Veredeln nach einem der Ansprüche 1 bis 3, bei dem die Veredelungszusammensetzung ein Haftmittel enthält, um die Haftung der Zusammensetzung auf einem Substrat zu erleichtern.

5. Verfahren zum Drucken und Veredeln nach einem der Ansprüche 1 bis 3, bei dem die Veredelungszusammensetzung eine wässrige oder organische Lösung von Silberionen enthält.

6. Verfahren zum Drucken und Veredeln nach einem der Ansprüche 1 bis 3, bei dem die Veredelungszusammensetzung eine Hydrosol- oder Organosolsuspension von Silberionenteilchen enthält.

7. Verfahren zum Drucken und Veredeln nach Anspruch 4, wobei das Haftmittel eines oder mehrere aus der Gruppe umfasst, die ein Naturharz, ein Kunstharz, Kolophonium, ein Polymer, ein Monomer, ein Bindemittel, einen Filmbildner, einen Klebstoff, eine Deckschicht, einen Lack, Acryl, modifiziertes Öl, Leinöl, Polyurethan, Proteinharz, Schellack, Acrylat und Epoxid umfasst.

8. Verfahren zum Drucken und Veredeln nach einem der Ansprüche 1 bis 3, bei dem die Veredelungszusammensetzung ein Lösungsmittel enthält.

9. Verfahren zum Drucken und Veredeln nach Anspruch 8, wobei das Lösungsmittel eines oder mehrere aus der Gruppe umfasst, die Wasser, Alkohol, einen Ester, Pflanzenöl und ein Erdöldestillat umfasst.

10. Verfahren zum Drucken und Veredeln nach einem der vorhergehenden Ansprüche, wobei die Veredelungszusammensetzung einen oder mehrere Zusatzstoffe aus der Gruppe enthält, die ein Metall, ein Mattierungsmittel, ein Wachs, ein Glanzmittel, einen Oberflächenmodifikator, ein Pigment, einen Farbstoff, einen Trockner, einen Vernetzer, einen Beschleuniger und ein Antirutschmittel umfasst.

11. Verfahren zum Drucken und Veredeln nach einem der vorhergehenden Ansprüche, wobei die Veredelungszusammensetzung eine Konzentration von 0,1 % eines Silberadditivpulvers in einer Beschichtung auf Ölbasis umfasst.

12. Verfahren zum Drucken und Veredeln nach einem der Ansprüche 1 bis 10, bei dem die Veredelungszusammensetzung eine 1,0 %ige Konzentration eines flüssigen Silberadditivs in einer Beschichtung auf Wasserbasis enthält.

13. Verfahren zum Drucken und Veredeln nach einem der vorhergehenden Ansprüche, wobei die antibakterielle Aktivität die Anforderungen von JIS Z2801/ISO22196:2007 für MRSA und E.coli erfüllt oder übertrifft.

14. Verfahren zum Drucken und Veredeln nach einem der vorhergehenden Ansprüche, wobei das Substrat eines oder mehrere aus der Gruppe umfasst, die Papier, Pappe, Karton und ein Polymer umfasst.

## Revendications

1. Procédé d'impression et de finition sur un substrat, le procédé comprenant les étapes suivantes, réalisées en un seul passage du substrat à travers une presse d'impression :
une étape d'impression dans laquelle une ou plusieurs couches d'impression sont déposées sur l'une ou les deux faces du substrat ; et une étape de finition dans laquelle une composition de finition liquide est appliquée sur toute la surface de l'une ou des deux faces du substrat en utilisant un système de chambre et de racle dans lequel la composition de finition liquide est pompée vers une chambre et remplit les cellules d'un rouleau de transfert, une racle servant à écumer l'excès de composition de finition liquide avant le transfert de la composition de finition liquide sur le substrat, et est durcie ou séchée pour former une couche protectrice extérieure continue sur le substrat, la composition de finition comprenant un additif antibactérien qui confère aux articles imprimés des propriétés antibactériennes.

2. Procédé d'impression et de finition selon la revendication 1, dans lequel l'étape d'impression est réalisée sur une première zone du substrat en même temps que l'étape de finition est réalisée sur une seconde zone du substrat.

3. Procédé d'impression et de finition tel que revendiqué dans l'une quelconque des revendications 1 à 2, dans lequel le procédé d'impression est l'un ou plusieurs du groupe comprenant un procédé lithographique, un procédé analox, un procédé offset en bande et un procédé Tresu.

4. Procédé d'impression et de finition selon l'une quelconque des revendications 1 à 3, dans lequel la composition de finition comprend un agent d'adhésion pour faciliter l'adhésion de la composition à un substrat.

5. Procédé d'impression et de finition selon l'une quelconque des revendications 1 à 3, dans lequel la composition de finition comprend une solution aqueuse ou organique d'ions argent.

6. Procédé d'impression et de finition selon l'une quelconque des revendications 1 à 3, dans lequel la composition de finition comprend une suspension hydrosol ou organosol de particules d'ions argent.

7. Procédé d'impression et de finition selon la revendication 4, dans lequel l'agent d'adhérence comprend un ou plusieurs éléments du groupe comprenant une résine naturelle, une résine synthétique, une colophane, un polymère, un monomère, un liant, un agent filmogène, un adhésif, une couche de finition, un vernis, un acrylique, une huile modifiée, une huile de lin, un polyuréthane, une résine protéique, une gomme-laque, un acrylate et un époxy.

8. Procédé d'impression et de finition selon l'une quelconque des revendications 1 à 3, dans lequel la composition de finition comprend un solvant.

9. Procédé d'impression et de finition selon la revendication 8, dans lequel le solvant comprend un ou plusieurs éléments du groupe comprenant l'eau, un alcool, un ester, une huile végétale et un distillat de pétrole.

10. Procédé d'impression et de finition selon l'une quelconque des revendications précédentes, dans lequel la composition de finition comprend un ou plusieurs additifs quelconques du groupe comprenant un métal, un agent de matage, une cire, un agent de brillance, un modificateur de surface, un pigment, un colorant, un siccatif, un agent de réticulation, un accélérateur et un agent antidérapant.

11. Procédé d'impression et de finition selon l'une quelconque des revendications précédentes, dans lequel la composition de finition comprend une concentration de 0,1% de poudre d'additif d'argent dans un revêtement à base d'huile.

12. Procédé d'impression et de finition selon l'une quelconque des revendications 1 à 10, dans lequel la composition de finition comprend une concentration de 1,0% d'additif liquide à l'argent dans un revêtement à base d'eau.

13. Procédé d'impression et de finition selon l'une quelconque des revendications précédentes, dans lequel l'activité antibactérienne satisfait ou dépasse les exigences de JIS Z2801/ISO22196:2007 pour MRSA et E.coli.

14. Procédé d'impression et de finition selon l'une quelconque des revendications précédentes, dans lequel le substrat comprend un ou plusieurs éléments du groupe comprenant le papier, la carte, le carton et un polymère.
